# EUROPEAN PATENT SPECIFICATION

(11) **EP 0 465 008 B1**
(45) Date of publication and mention of the grant of the patent: **08.05.1996**
(21) Application number: 91305063.9
(22) Date of filing: 05.06.1991
(51) Int. Cl.: G01N 33/573, C12P 21/08, C12N 5/20, C07K 16/40

(54) **A monoclonal antibody recognizing human pancreatic prophospholipase A2**
Ein monoklonaler Antikörper gegen humane, pankreatische Prophospholipase A2
Un anticorps monoclonal contre prosphopholipase A2 humain pancréatique

(30) Priority: 05.07.1990 JP 179137/90
(43) Date of publication of application: 08.01.1992
(73) Proprietor: SHIONOGI SEIYAKU KABUSHIKI KAISHA, Osaka 541 (JP)
(72) Inventor: Misaki, Atsushi, Osaka-shi, Osaka (JP); Kono, Masao, Ibaraki-shi, Osaka (JP)
(74) Representative: Jones, Michael Raymond

(56) References cited:
- EP-A- 0 287 397
- WO-A-88/01059
- THE JOURNAL OF BIOCHEMISTRY vol. 94, no. 1, July 1983, TOKYO JP pages 137 - 147, J. NISHIJIMA etal.
- THE JOURNAL OF CLINICAL BIOCHEMISTRY AND NUTRITION vol. 11, no. 2, February 1991, pages 91 - 100 A.MISAKI ET AL. 'Method for simultaneous detection of pancreatic phospholipase A2 and prophospholipase A2 in human sera and its clinical significance'

## Description

The present invention relates to a monoclonal antibody recognizing human prophospholipase A₂ (hereinafter referred to as proPLA₂mAb), a method for the assay of human pancreatic prophospholipase A₂ using the monoclonal antibody, and a hybridoma producing the monoclonal antibody.

Human pancreatic phospholipase A₂ (EC 3.1.1.4; in the following account, phospholipase A₂ is referred to as PLA₂) is formed from prophospholipase A₂ (hereinafter referred to as proPLA₂) which is enzymatically inactive and is produced by the acinar cells of the pancreas. After production in the pancreas, the proPLA₂ is secreted into the duodenum, and is therein decomposed by trypsin and converted into the active form PLA₂. Both PLA₂ and proPLA₂ are known to be present in blood serum. Serum levels of PLA₂ have previously been measured by enzymatic methods [Zieve L and Vogel WC, J. Lab. Clin. Med., 57, 586(1961); Doizaki WM and Zieve L, J. Lab. Clin. Med., 67, 108 (1966); Hashihira S et al., Jpn. Arch. Intern. Med., 24, 243 (1977); and Schröder T et al., Scand. J. Gastroent., 15, 633 (1980)]. Moreover, immunological assays of this enzyme have also been performed [Nishijima J et al., J. Biochem., 94, 137 (1983); and Eskola JU et al., Clin. Chem., 29, 1777 (1983)]. Elevated serum levels of PLA₂ have been reported as occurring in cases of acute pancreatitis (ibid.). Furthermore, monoclonal antibodies have been prepared against PLA₂ (Japanese Laid-Open Patent Publication No. 63-258898), and radioimmunoassays employing these antibodies have suggested that nearly all of the said enzyme in blood serum of healthy humans assumes the form of proPLA₂, while the quantity of active PLA₂ in healthy serum is extremely minute [Misaki et al., Ann. Jpn. Clin. Chem. Soc., 27, 109 (1987)]. The aforesaid report of Misaki et al. also suggests that proPLA₂ is transformed into active PLA₂ in the serum of some patients with acute pancreatitis. Büchler M et al. [Klin. Wochenschr, 67, 186 (1989)] report that both immunologically active PLA₂ levels (reflecting the quantities of both PLA₂ and proPLA₂) and PLA₂ catalytic activity (reflecting only the quantity of PLA₂, since proPLA₂ is inactive) are elevated in the serum of patients with necrotic pancreatitis, whereas only immunologically active PLA₂ levels are elevated in edematous pancreatitis patients.

Serum levels of PLA₂ and proPLA₂ are known to increase not only in pancreatitis, as described above, but also, according to other research results, in cancer, articular rheumatism, external injuries and other disorders. Therefore, precise quantitation of serum levels of PLA₂ and proPLA₂ is regarded as an important datum with respect to the diagnosis and monitoring of patients with the aforesaid diseases. As mentioned above, monoclonal antibodies against PLA₂ have previously been developed. However, these monoclonal antibodies are known to display considerable cross-reactivity with proPLA₂. Consequently, for example, radioimmunoassays performed with these antibodies cannot yield precise measurements of PLA₂ levels. From the viewpoint of diagnosis and clinical monitoring of the aforesaid disorders, the development of monoclonal antibodies which permit accurate and specific measurement of serum levels of both PLA₂ and proPLA₂ is an important desideratum.

According to one aspect of the present invention there is provided a monoclonal antibody specifically recognizing human pancreatic prophospholipase A₂, wherein said antibody is monoclonal antibody PR414 produced by hybridoma PR414 (FERM BP-2978).

In a preferred embodiment, the cross-reactivity of the monoclonal antibody with human pancreatic phospholipase A₂ is less than 1%.

According to another aspect of the present invention there is provided a method for the assay of human pancreatic prophospholipase A₂ of this invention comprising the steps of,
(a) adding a prescribed quantity of labeled human pancreatic prophospholipase A₂ to a sample containing human pancreatic prophospholipase A₂ analyte,
(b) adding the above-mentioned monoclonal antibody to the mixture obtained in process (a), thereby forming an antibody-antigen complex, and
(c) calculating the quantity of human pancreatic prophospholipase A₂ in the sample from the result of measurement of the quantity of label bound in the complex obtained in process (b).

Also provided are hybridomas which produce the above-mentioned monoclonal antibodies.

Thus, the invention described herein makes possible the objectives of:
(1) providing a monoclonal antibody against proPLA₂ which permit precise measurement of human serum levels of proPLA₂, and
(2) providing a method for the assay of proPLA₂ by the use of the aforesaid monoclonal antibody.

For a better understanding of the invention, and to show how the same may be carried into effect, reference will now be made to the accompanying drawings, in which:
Figure 1 is a graph showing the calibration curve for measurement of proPLA₂ by radioimmunoassay by the use of the monoclonal antibody of the present invention.
Figures 2(A) and 2(B) are graphs showing the elution profiles obtained when the blood sera of a healthy human and a pancreatitis patient, respectively, were subjected to ion exchange chromatography.

The present invention comprises hybridomas producing the aforesaid monoclonal antibody.

The monoclonal antibody of this invention which specifically recognizes proPLA₂ can be prepared by the use of a hybridoma in accordance with conventional procedures. For example, by the use of the method of Grataroli, R et al. [Biochimie, 63, 677 (1981)], proPLA₂ is extracted and purified from pancreatic juice, and used to immunize mice or other suitable animals. Then, splenic or other antibody-producing cells are fused with myeloma cells to obtain hybridomas. Cells derived from mouse myeloma, for example, can be used as the aforesaid myeloma cells. The aforesaid cell fusion can be effected, for example, by a conventional procedure using polyethylene glycol. Then, from the fused cells so obtained, monoclonal antibodies against proPLA₂ can be obtained by screening. This screening can be accomplished by conventional enzyme-linked immunosorbent assay (ELISA) methods. For example, purified proPLA₂ is adsorbed onto the solid phase, and this is brought into contact with the supernate of the hybridoma culture; if antibodies recognizing proPLA₂ are present in this supernate, then these antibodies bind to the proPLA₂. Next, an appropriate enzyme-labeled antibody, for example, mouse anti-IgG antibody, is allowed to act and binds to the anti-proPLA₂ antibodies. Thus, detection of the enzyme label permits screening for the antibodies recognizing proPLA₂.

Next, the hybridomas producing these antibodies are further screened for those producing antibodies displaying minimal cross-reactivity with PLA₂, that is, monoclonal antibodies specifically recognizing proPLA₂. For example, by means of ELISA or RIA (radioimmunoassay) using PLA₂ and proPLA₂, the hybridomas can be screened for monoclonal antibodies which bind specifically to proPLA₂ and display almost no binding to PLA₂. Thus, hybridomas producing monoclonal antibodies specifically recognizing proPLA₂ are obtained. In the present invention, eight hybridoma cell lines, designated as PR304, PR306, PR307 PR312, PR314, PR318, PR414, and PR424, were obtained in the aforesaid manner. Among these cell lines, PR414 has been deposited with the Fermentation Research Institute Agency for Industrial Science and Technology, 1-3, Higashi 1-chome, Tsukuba-shi, Ibaraki-ken, Japan on June 21, 1990 under Accession number FERM BP-2978 in accordance with the Budapest Treaty.

Under the conditions of conventional methods of cultivation, the monoclonal antibodies of the present invention are secreted into the supernate. Moreover, by transplanting the aforesaid hybridomas into the peritoneal cavities of mice, ascitic fluid containing high concentrations of the said monoclonal antibodies can be obtained. The use of these monoclonal antibodies permits accurate measurement of proPLA₂ or PLA₂. This measurement can be performed by generally known RIA or enzyme immunoassay procedures. For example, proPLA₂ can be measured by the following method (competitive assay). First, a prescribed quantity of labelled (e.g., radiolabeled) proPLA₂ is added to a sample containing a known quantity of proPLA₂, then, the aforesaid monoclonal antibody specifically recognizing proPLA₂ is added to the mixture. Thus, labeled or unlabeled proPLA₂ binds to each monoclonal antibody molecule, thereby forming antibody-antigen complexes. Then, for example, Immunobead (Bio-Rad Co.) was added to the sample, thus binding the said complexes. Then, the bound materials are separated from the reaction mixture in the form of pellets, and the quantity of label on the pellets is measured. In this manner, a calibration curve is prepared by varying the quantity of proPLA₂ in the sample. By the use of this calibration curve, unknown quantities of proPLA₂ can be measured in a similar manner.

The PLA₂ content of blood serum containing both proPLA₂ and PLA₂ can be measured in the following manner. First, anti-PLA₂ monoclonal antibody having known cross-reactivity (x%) with proPLA₂ is prepared. The quantity of proPLA₂ in a sample containing unknown concentrations of both PLA₂ and proPLA₂ can be measured by the aforesaid method; let A denote the molar concentration of proPLA₂ determined in this manner. Next, the same measurement is performed by the use of the aforesaid cross-reactive anti-PLA₂ monoclonal antibody in place of anti-proPLA₂ monoclonal antibody; let B denote the molar concentration so obtained. Since B is the sum of the value corresponding to PLA₂ and x% of the value corresponding to proPLA₂, the concentration of PLA₂ is expressed by the formula (B - (X/100)A). Thus, by the use of the monoclonal antibody of the present invention, serum levels of both PLA₂ and proPLA₂ can be accurately determined.

### EXAMPLES

Next, the present invention will be explained in further detail with reference to specific examples. However, these examples are merely illustrative and by no means limit the scope of the present invention.

### EXAMPLE 1

### (1) Preparation of PLA₂ and proPLA₂

PLA₂ was extracted and purified from pancreatic juice in accordance with the method of Nishijima et al. [J. Biochem., 94, 137 (1983)]. ProPLA₂ was extracted and purified from pancreatic juice in accordance with the method of Grataroli et al. [Biochimie, 63, 677 (1981)], except that CM-Sepharose (registered trademark) chromatography was performed twice. The purities of both PLA₂ and proPLA₂ were verified by amino acid analysis.

### (2) Preparation of monoclonal antibody against PLA₂ (anti-PLA₂mAb)

Among the varieties of anti-PLA₂mAb obtained by the method of Misaki et al. (Japanese Laid-Open Patent Publication No. 63-258898), that designated as mAb1008 was used in the present invention. The affinity constant of mAb1008 was 4x10¹¹M⁻¹, and the cross-reactivity of this antibody with proPLA₂ was 49%.

### (3) Preparation of anti-proPLA₂ mAb

Female BALB/c mice were immunized with pro-PLA₂ in the following manner. First, 25 µg of proPLA₂ emulsified with Freund's complete adjuvant was subcutaneously injected into the mice three times, at intervals of 3 weeks. In addition, physiological saline containing 50 µg of proPLA₂ was administered intraperitoneally 4 days prior to the collection of splenic cells from these mice.

Then, splenic cells were collected from the aforesaid mice and fused with mouse myeloma cells (P3-Ns1-1-Ag4-1) by the use of 50% polyethylene glycol (molecular weight 4000, Merck) in accordance with the method of Galfre and Milstein [Methods Enzymol., 73, 3 (1981)]. Next, the fused cells were suspended in HAT medium and inoculated into Costar 96-well Tissue Culture Cluster (trade name). Then, the supernates of the cultures in a total of 1503 wells wherein the hybridomas had been grown were screened by the following ELISA procedure.

The wells of 96-well Immunoplates (Nunc Co.) were coated overnight at 4°C with 10 ng of proPLA₂ or 10 ng of PLA₂ in 50 µl of 0.05 M sodium bicarbonate buffer, pH 8.5. Next, the wells were blocked with 0.2% BSA-PBS and washed with 0.5% Tween (registered trademark) 80-PBS. Twenty-five microliter aliquots of supernate from each of the aforesaid 1503 hybridoma cultures were added to the aforesaid proPLA₂-coated wells as well as PLA₂-coated wells, after which these plates were incubated at 37°C for 1 hour. The antibody which bound to the proPLA₂ or PLA₂ on the wells was quantitated by means of a Vectastain ABC kit (Vector Co.), with the use of ABTS as a color-producing agent. In the process of the measurement, the reaction mixture was left at room temperature, and allowed to color. Twelve minutes after the agent was added, the mixture was measured at 415 nm using a Corona MTP-32 microplate photometer. Wells displaying a large absorbance (greater than 1.0) in the ELISA procedure using proPLA₂-coated plates and a small absorbance (less than 0.5) when using the PLA₂-coated plates were selected. In this manner, hybridomas producing anti-proPLA₂ mAb (i.e., binding to proPLA₂ and having very low cross-reactivity with PLA₂) were obtained (31 wells).

Some of these ELISA positive hybridomas were cloned immediately after screening to further proliferation and these hybridomas were frozen subsequently. The approximate affinity of the antibodies produced by the selected hybridomas for proPLA₂ was estimated using the supernatant recovered when these hybridomas were frozen. The aforesaid affinity was determined by the competitive RIA procedure described in the section entitled "Description of the Preferred Embodiment". Hybridomas producing antibodies displaying high affinity were cloned, if necessary.

In this manner, eight mouse hybridoma cell lines producing anti-proPLA₂mAb (designated as PR304, PR306, PR307, PR312, PR314, PR318, PR414, and PR424) were established. The culture supernatants were used as the source of anti-proPLA₂mAb in the Examples of the present invention.

The characteristics of the anti-proPLA₂mAb produced by aforesaid eight hybridoma cell lines are shown in Table 1. The immunoglobulin class as determined by the Ouchterlony diffusion method was IgG₁ in every case. The affinity constants were determined from the Scatchard plots [Scatchard G, Ann. N. Y. Acad. Sci., 51, 660 (1949)].

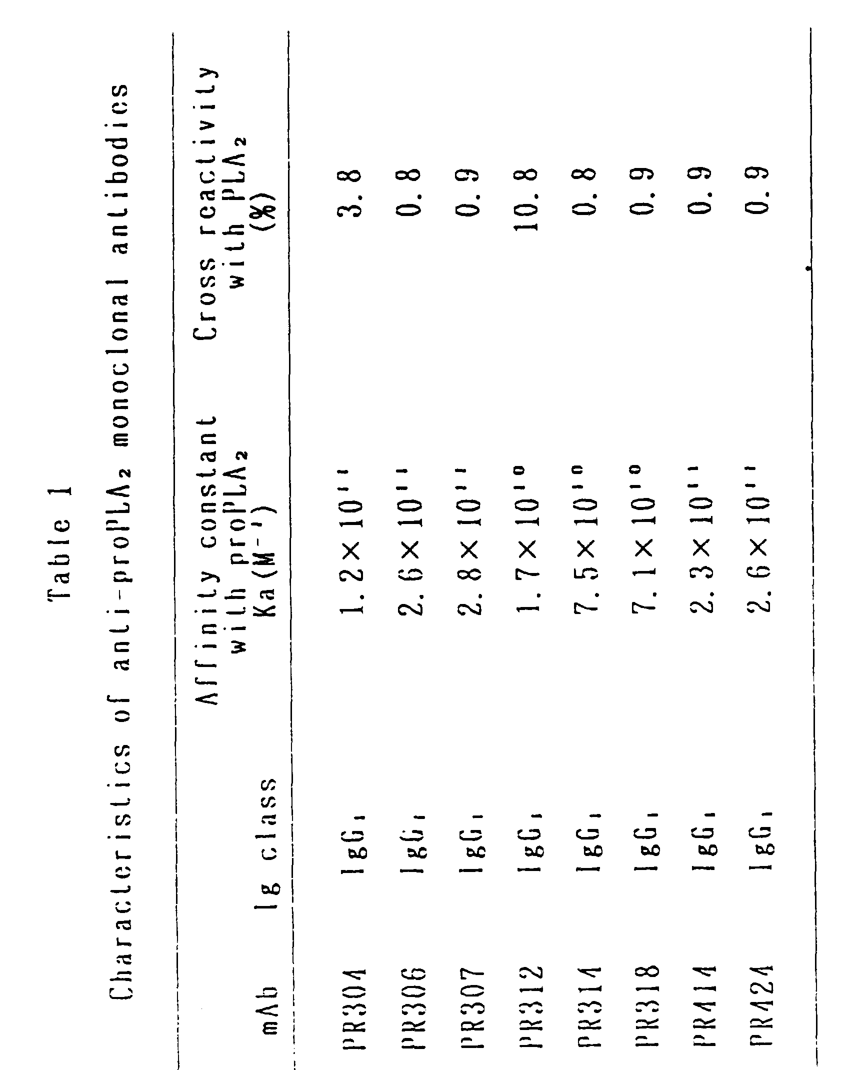

As is clear from Table 1, all of these monoclonal antibodies display high affinity for proPLA₂, with affinity constants ranging from 1.7x10¹⁰M⁻¹ to 2.8x10¹¹M⁻¹. Cross-reactivity with PLA₂ was estimated from the 50% inhibitory ratio of the calibration curve determined by competitive RIA by the use of the same procedure as that described above. MAbPR304 and mAbPR312 displayed significant cross-reactivity with PLA₂, but the cross-reactivity for the other six varieties of mAb was less than 1%.

Among the aforesaid eight varieties of anti-proPLA₂ monoclonal antibodies, mAbPR414 was used in the following experiments. The cross-reactivity of mAbPR414 with PLA₂ is 0.9%.

### (4) Assays of proPLA₂ and PLA₂

RIA for the measurement of proPLA₂ was performed with the aforesaid proPLA₂mAbPR414 (i.e., using the culture supernatant diluted 320-fold with assay buffer solution; the cross-reactivity of anti-proPLA₂ mAbPR414 with PLA₂ was 0.9%) and ¹⁵I-labeled proPLA₂. RIA for the measurement of PLA₂ was performed with the aforesaid anti-PLA₂mAb1008 (i.e., using ascitic fluid diluted 300,000-fold with assay buffer solution; the cross-reactivity of anti-PLA₂mAb1008 with proPLA₂ is 49%) and ¹⁵I-labeled PLA₂. The assay buffer solution used for RIA was a 0.01M PBS (pH 7.4) containing 1 mM EDTA, 0.01% NaN₃, and 0.2% BSA. Both ¹⁵I-proPLA₂ and ¹⁵I-PLA₂ were prepared by the chloramine T method.

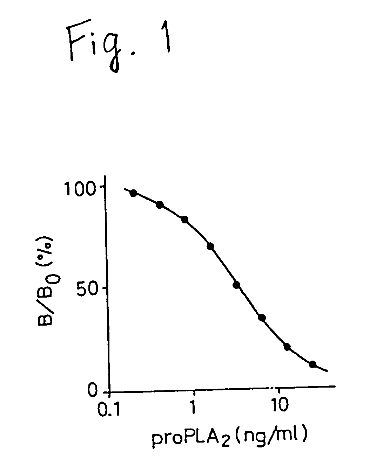

Calibration curves for proPLA₂ and PLA₂ were prepared by the following procedure. First, 100 µl of a standard solution that contains proPLA₂ or PLA₂ in a predetermined amount, 100 µl of a solution that contains ¹⁵I-proPLA₂ or ¹⁵I-PLA₂ (40,000 cpm), 100 µl of mAb solution (i.e., the aforesaid diluted supernatant containing mAb PR414, or the aforesaid diluted ascitic fluid containing mAb1008), and 200 µl of assay buffer solution, were mixed in a polystyrene tube, after which the mixture was incubated overnight at room temperature. Then, 100 µl of Immunobead (1 mg anti-mouse immunoglobulin/ml assay buffer, Bio-Rad Co.) was added, and the mixture was left undisturbed for 1 hour at room temperature. Next, the tube was centrifuged at 3,000 rpm for 5 minutes, and the radioactivity of the pellet so obtained was measured. Figure 1 shows the calibration curve representing the relation between the measured radioactivity and the proPLA₂ concentration of the standard solution. The ordinate B/B₀ of the graph in Figure 1 is the ratio between the measured radioactivity B corresponding to the respective standard solutions and the radioactivity B₀ corresponding to a zero concentration of proPLA₂.

The sensitivity of the calibration curve, that is, the lowest measurable concentration (i.e., the concentration of proPLA₂ corresponding to 90% inhibition) was 0.4 ng/ml, while the concentration of proPLA₂ corresponding to 50% inhibition was 3.2 ng/ml. The calibration curve for RIA of PLA₂ (not shown) revealed that the concentration of PLA₂ corresponding to 90% inhibition was 0.4 ng/ml, while that corresponding to 50% inhibition was 3.0 ng/ml.

In the following examples, the aforesaid assays were performed in the same manner, using sample liquids containing human serum, or eluates obtained by ion exchange chromatography of human serum in place of the aforesaid standard solutions of various concentrations of proPLA₂ or PLA₂. The concentrations of proPLA₂ and PLA₂ in these sample liquids was determined by using the calibration curves for proPLA₂ and PLA₂, respectively, prepared in the manner indicated above.

### EXAMPLE 2

### Assay of serum levels of proPLA₂ and PLA₂ by RIA using anti-proPLA₂ mAb and anti-PLA₂ mAb

Blood sera of the following healthy humans and patients were assayed by the same RIA procedure described in Example 1 above. The subjects comprised three healthy individuals, three pancreatitis patients (No. 1: patient with moderate pancreatitis following contrast radiography of the pancreatic duct; No. 2: patient with chronic pancreatitis in a period of exacerbation; No. 3: patient with acute necrotizing pancreatitis), one patient with pancreatic carcinoma, and one patient with chronic renal insufficiency. The results of the RIA studies are shown in Table 2.

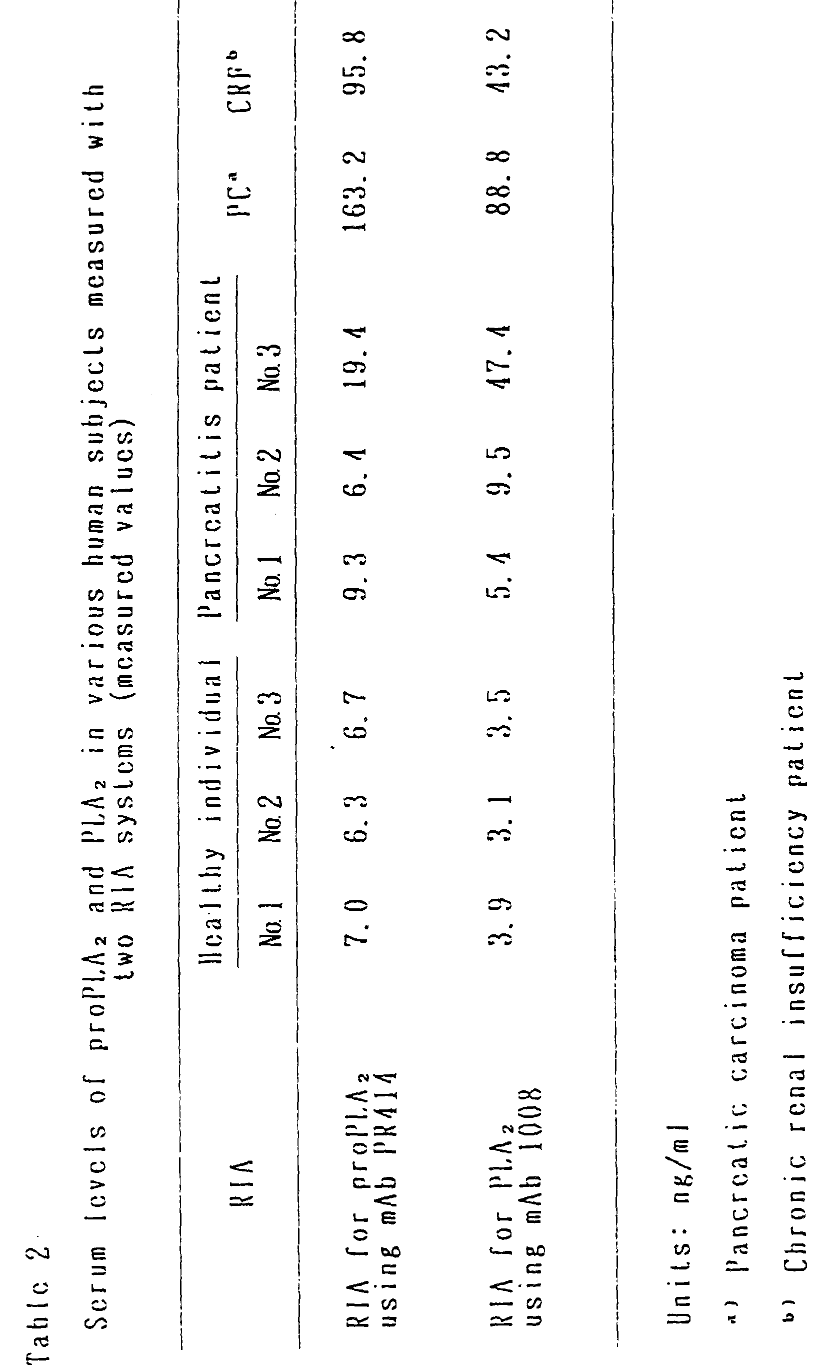

The serum levels of proPLA₂ shown in Table 2 are considered to agree almost completely with the true serum concentrations of proPLA₂. On the other hand, since the monoclonal antibody used for the assay of PLA₂ displayed 49% cross-reactivity with proPLA₂, the serum levels of PLA₂ shown in Table 2 are proportionately higher than the actual values. According to the data in this table, the measured levels of both proPLA₂ and PLA₂ in the sera of the pancreatitis, pancreatic carcinoma and chronic renal insufficiency patients were higher than the corresponding values for the healthy individuals, except for pancreatitis patient No. 2. The measured levels of proPLA₂ were higher (by a factor of approximately 2) than those of PLA₂ in the healthy individuals as well as the pancreatic carcinoma patient and the chronic renal insufficiency patient. Furthermore, in two of the three pancreatitis patients (Nos. 2 and 3), the measured levels of PLA₂ were higher than those of proPLA₂.

The aforesaid measured proPLA₂ values were directly recorded as the serum concentrations of proPLA₂. Next, taking into account the aforesaid cross-reactivity of mAb1008, the serum concentrations of PLA₂ were calculated on the basis of the measured PLA₂ values. The serum levels of proPLA₂ and PLA₂ determined in this manner as well as the ratios of proPLA₂ and PLA₂ concentrations are shown in Table 3.

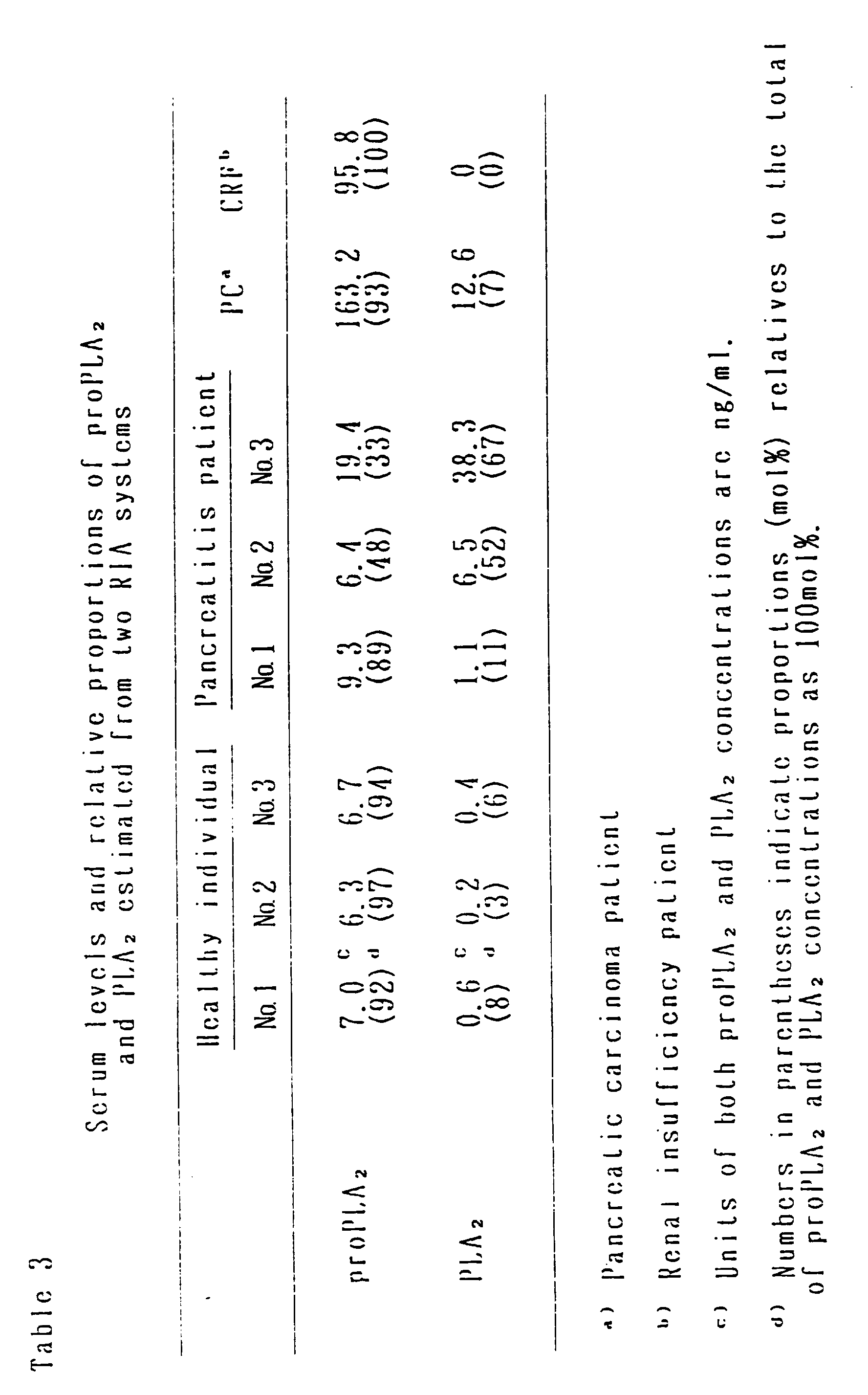

### EXAMPLE 3

### Isolation of serum proPLA₂ and PLA₂ by ion exchange chromatography and measurement by RIA

ProPLA₂ and PLA₂ in human blood serum were isolated by ion exchange chromatography using S-Sepharose (registered trademark) (pharmacia), after which measurement was performed by RIA. As the buffer solution for chromatography, 10 mM Tris-HCl (pH 7.0) containing 0.05% CHAPS was used and the entire chromatographic process was performed at room temperature.

First, proPLA₂ and PLA₂ standards (dissolved in chromatographic buffer solution containing 0.2% BSA), prepared for the purposes of column calibration, were injected into an S-Sepharose (registered trademark) column (bed capacity 2 ml) equilibrated with the aforesaid chromatographic buffer solution. Then, the column was eluted with 40 ml of the chromatographic buffer solution under a 0-0.2 M linear gradient of sodium chloride, at a flow rate of approximately 12 ml/hour. After completion of the linear gradient elution, the column was again eluted with 0.5 M sodium chloride. One milliliter aliquots of all the eluates from the column were collected in polypropylene tubes containing 100 µl of 2% BSA-PBS. ProPLA₂ and PLA₂ standards were recovered from the S-Sephadex column at high recovery rates. The peak fractions were fraction 19 for proPLA₂ and fraction 26 for PLA₂.

Next, human sera (i.e., the same lot of eight sample liquids as was used in Example 2), diluted 5-fold with the aforesaid chromatographic buffer, were injected into the column and separation was performed by the same procedure.

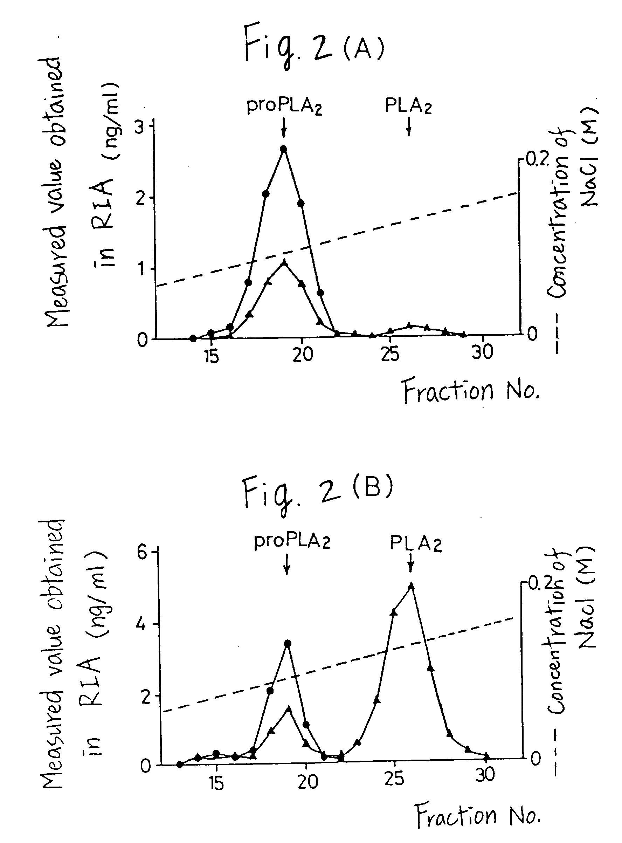

The proPLA₂ and PLA₂ content of the various eluted fractions of the aforesaid standards and human sera were measured by RIA using the same procedure as in Example 2. Except for a small peak which appeared in the vicinity of the fraction 15 for the pancreatitis patients, all of the immunoreactive components were eluted with the same retention volumes as the standards. No immunoreactivity was detected in either the initial fraction or the 0.5 M sodium chloride fraction. Figures 2(A) and 2(B) show the elution profiles for the sera of one of the healthy individuals (No. 1) and one of the pancreatitis patients (No. 3), respectively. In these figures, the black dots represent concentrations of proPLA₂ values measured by RIA using mAb PR414, while the black triangles represent concentrations of PLA₂ measured by RIA using mAb1008. The concentrations and relative proportions of proPLA₂ and PLA₂ in the sera of the eight subjects, calculated from the chromatographic peak regions, are shown in Table 4.

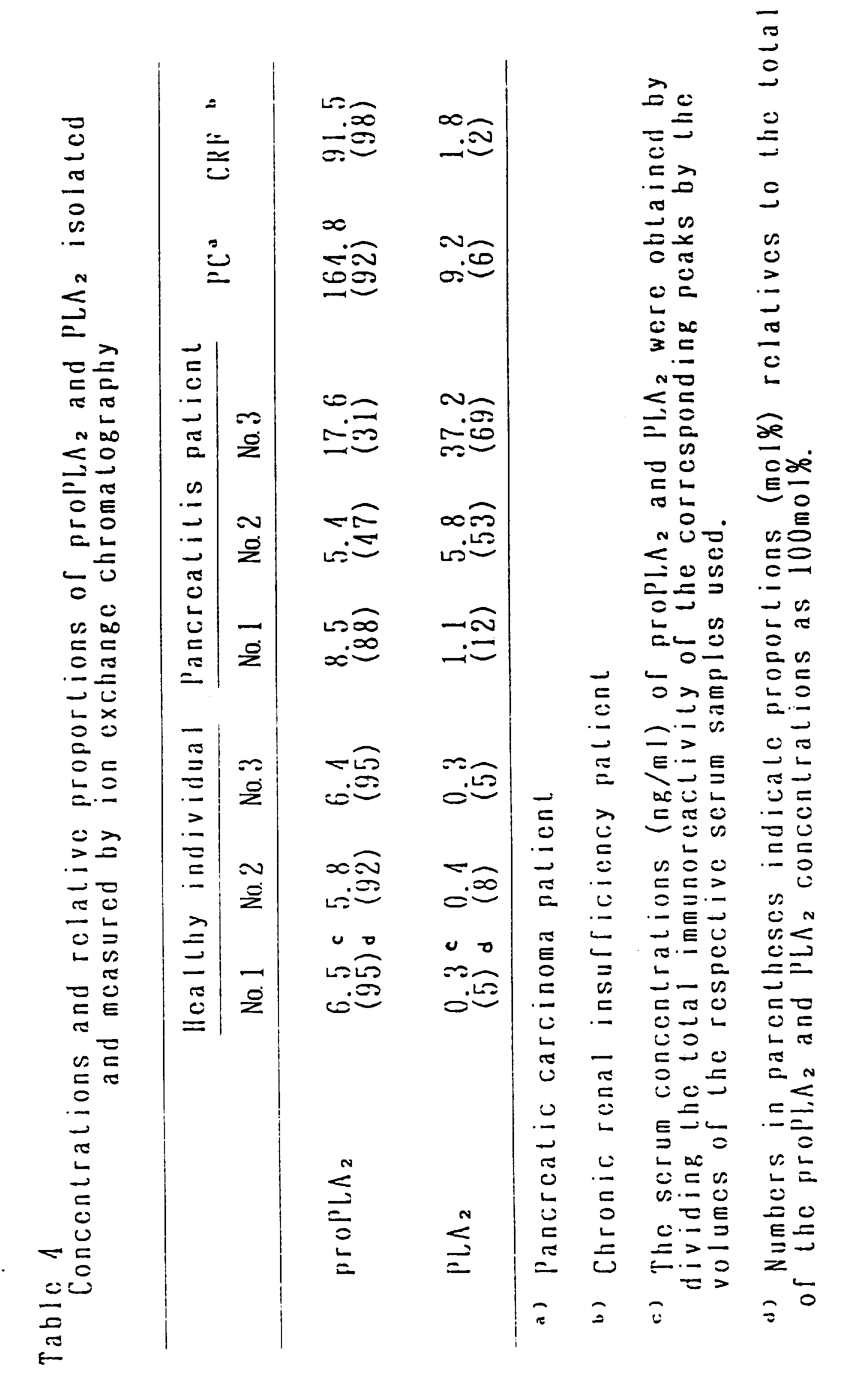

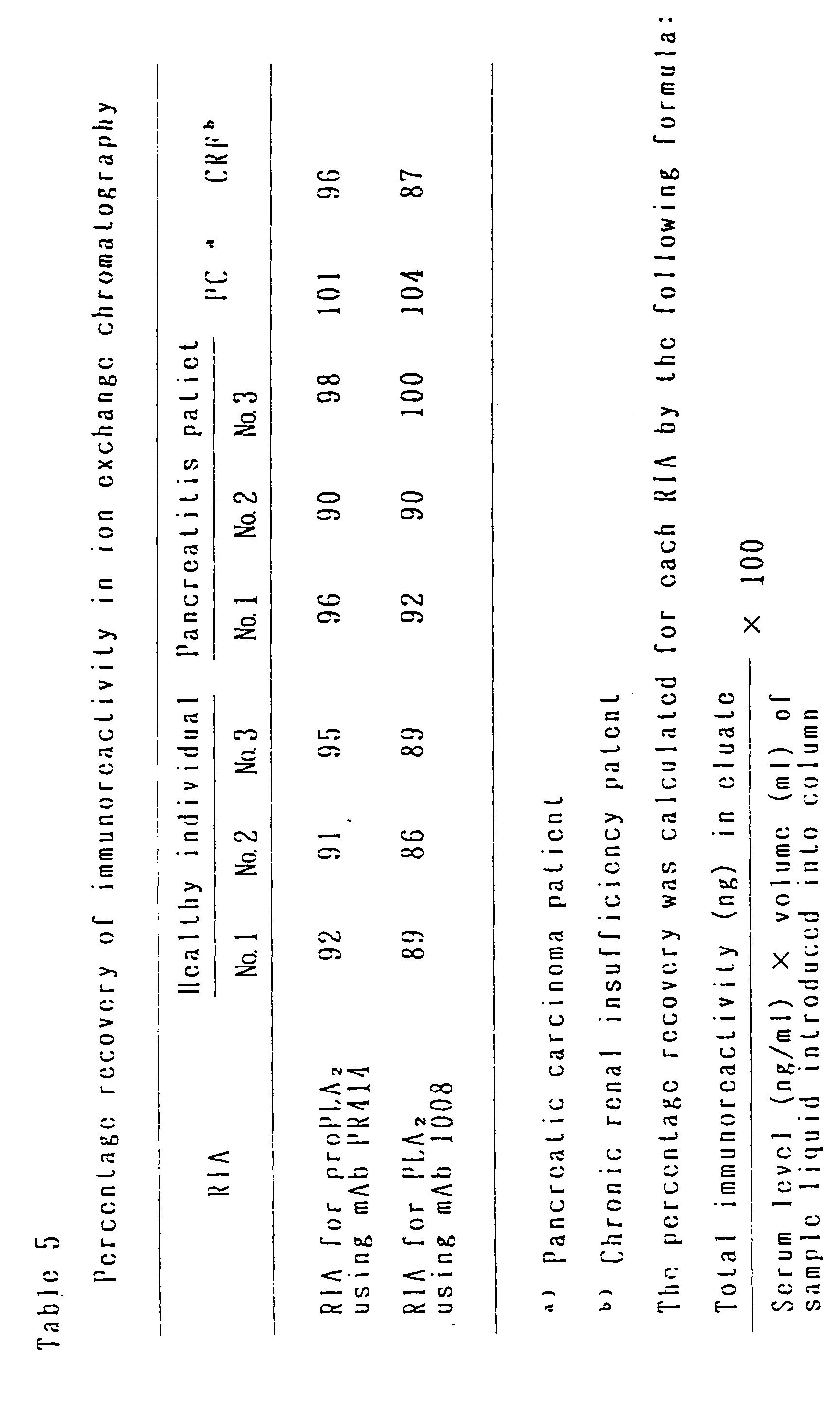

Table 5 shows the recovery rate of immunoreactivity of proPLA₂ and PLA₂ by ion exchange chromatography, as determined by RIA. As is seen from the table, the recovery rate for every tested human serum was approximately 100% (86% - 104%). This indicates that RIA for proPLA₂ using mAb PR414 measures the serum level of proPLA₂ only, while RIA for PLA₂ using mAb1008 measures the serum level of PLA₂ plus the quantity corresponding to cross-reactivity with proPLA₂. The serum levels of proPLA₂ and PLA₂ calculated by means of the monoclonal antibody of the present invention, shown in Table 3, are nearly identical to those shown in Table 4. Therefore, it is recognized that the use of the monoclonal antibody of the present invention permits extremely precise measurement of both proPLA₂ and PLA₂.

The data in Tables 2-4 indicate that PLA₂ in the serum of healthy humans assumes the form of the enzymatically inactive proPLA₂ predominantly. This indicates that proPLA₂, which is synthesized in the pancreas and diffuses into the bloodstream, exists without activation in normal serum. Consequently, the cytotoxicity of PLA₂ is not manifested in healthy individuals. In the patient with comparatively moderate pancreatitis (No. 1), the serum level of proPLA₂ was higher than that of PLA₂, however, in the patient with chronic pancreatitis during a period of exacerbation (No. 2) as well as the patient with acute necrotizing pancreatitis (No. 3), the serum concentration of PLA₂ was higher than that of proPLA₂. This suggests that the relative proportions of proPLA₂ and PLA₂ reflect the degree of severity of pancreatitis. As regards the pancreatic carcinoma patient and the chronic renal insufficiency patient, the serum levels of both proPLA₂ and PLA₂ were extremely high. However, the relative proportions of proPLA₂ and PLA₂ were nearly the same as in the healthy subjects. In the renal insufficiency patient, this result may be attributed to impaired excretion of proPLA₂ and PLA₂ from the kidneys. This characteristic differs from those manifested in pancreatitis patients, and therefore could possibly be utilized for differential diagnosis.

## Claims (Claims for the following Contracting State(s): AT, BE, DK, FR, DE, NL, IT, LU, SE, CH, LI, GB)

1. A monoclonal antibody specifically recognizing human pancreatic prophospholipase A₂, wherein said antibody is monoclonal antibody PR414 produced by hybridoma PR414 (FERM BP-2978).

2. A monoclonal antibody of claim 1, wherein the cross-reactivity of said monoclonal antibody with human pancreatic phospholipase A₂ is less than 1%.

3. A method for the assay of human pancreatic prophospholipase A₂, comprising the steps of,
(a) adding a prescribed quantity of labeled human pancreatic prophospholipase A₂ to a sample containing human pancreatic prophospholipase A₂ analyte,
(b) adding a monoclonal antibody of claim 1 or 2 to the mixture obtained in process (a), thereby forming an antibody-antigen complex, and
(c) calculating the quantity of human pancreatic prophospholipase A₂ in said sample from the result of measurement of the quantity of label bound in the complex obtained in process (b).

4. A hybridoma which produces a monoclonal antibody of claim 1 or 2.

## Claims (Claims for the following Contracting State(s): ES, GR)

1. A monoclonal antibody specifically recognizing human pancreatic prophospholipase A₂, wherein said antibody is monoclonal antibody PR414 produced by hybridoma PR414 (FERM BP-2978).

2. A monoclonal antibody of claim 1, wherein the cross-reactivity of said monoclonal antibody with human pancreatic phospholipase A₂ is less than 1%.

3. A method for the assay of human pancreatic prophospholipase A₂, comprising the steps of,
(a) adding a prescribed quantity of labelled human pancreatic prophospholipase A₂ to a sample containing human pancreatic prophospholipase A₂ analyte,
(b) adding a monoclonal antibody of claim 1 or 2 to the mixture obtained in process (a), thereby forming an antibody-antigen complex, and
(c) calculating the quantity of human pancreatic prophospholipase A₂ in said sample from the result of measurement of the quantity of label bound in the complex obtained in process (b).

4. A hybridoma which produces a monoclonal antibody of claim 1 or 2.

5. A method for the production of a monoclonal antibody specifically recognizing human pancreatic prophospholipase A₂, wherein said antibody is monoclonal antibody PR414 produced by hybridoma PR414 (FERM BP-2978), comprising the steps of:
i) extracting and purifying prophospholipase A₂ from human pancreatic juice;
ii) immunising an animal with said prophospholipase A₂;
iii) isolating and fusing antibody-producing cells of said animal with myeloma cells to obtain hybridomas;
iv) screening said hybridomas for hybridomas which produce said monoclonal antibody; and
v) isolating said monoclonal antibody from said hybridomas which produce said monoclonal antibody.

6. A method as claimed in claim 5, wherein the cross-reactivity of said monoclonal antibody with human pancreatic phospholipase A₂ is less than 1%.

7. A method for the assay of human pancreatic prophospholipase A₂, comprising the steps of,
(a) adding a prescribed quantity of labelled human pancreatic prophospholipase A₂ to a sample containing human pancreatic prophospholipase A₂ analyte,
(b) adding a monoclonal antibody produced by the method of claim 5 or 6 to the mixture obtained in process (a), thereby forming an antibody-antigen complex, and
(c) calculating the quantity of human pancreatic prophospholipase A₂ in said sample from the result of measurement of the quantity of label bound in the complex obtained in process (b).

8. A method for the production of a hybridoma which produces the monoclonal antibody produced by the method of claim 5 or 6 to comprising the steps of:
i) extracting and purifying prophospholipase A₂ from human pancreatic juice;
ii) immunizing an animal with said prophospholipase A₂;
iii) isolating and fusing antibody-producing cells of said animal with myeloma cells to obtain hybridomas; and
iv) screening said hybridomas for hybridomas which produce said monoclonal antibody.

## Patentansprüche (Patentansprüche für folgende(n) Vertragsstaat(en): AT, BE, DK, FR, DE, NL, IT, LU, SE, CH, LI, GB)

1. Monoklonaler Antikörper, der humane pankreatische Prophospholipase A₂ in spezifischer Weise erkennt, wobei der Antikörper der von der Hybridoma-Zelle PR414 (FERM BP-2978) gebildete monoklonale Antikörper PR414 ist.

2. Monoklonaler Antikörper nach Anspruch 1, wobei die Kreuzreaktivität des monoklonalen Antikörpers gegenüber humaner pankreatischer Phospholipase A₂ weniger als 1 % beträgt.

3. Verfahren zur Analyse der humanen pankreatischen Prophospholipase A₂, bei dem man
(a) einer Probe, die humanes pankreatisches Prophospholipase A₂-Analyt enthält, eine vorgeschriebene Menge an markierter humaner pankreatischer Prophospholipase A₂ zugibt,
(b) der in der Verfahrensstufe (a) erhaltenen Mischung einen monoklonalen Antikörper gemäß Anspruch 1 oder 2 zugibt, wodurch ein Antikörper/Antigen-Komplex gebildet wird, und
(c) die Menge an humaner pankreatischer Prophospholipase A₂ in der Probe ausgehend von dem Ergebnis der Messung der Menge von an den in der Verfahrensstufe (b) erhaltenen Komplex gebundenem Marker berechnet.

4. Hybridoma-Zelle, welche einen monoklonalen Antikörper gemäß Anspruch 1 oder 2 bildet.

## Patentansprüche (Patentansprüche für folgende(n) Vertragsstaat(en): ES, GR)

1. Monoklonaler Antikörper, der humane pankreatische Prophospholipase A₂ in spezifischer Weise erkennt, wobei der Antikörper der von der Hybridoma-Zelle PR414 (FERM BP-2978) gebildete monoklonale Antikörper PR414 ist.

2. Monoklonaler Antikörper nach Anspruch 1, wobei die Kreuzreaktivität des monoklonalen Antikörpers gegenüber humaner pankreatischer Phospholipase A₂ weniger als 1 % beträgt.

3. Verfahren zur Analyse der humanen pankreatischen Prophospholipase A₂, bei dem man
(a) einer Probe, die humanes pankreatisches Prophospholipase A₂-Analyt enthält, eine vorgeschriebene Menge an markierter humaner pankreatischer Prophospholipase A₂ zugibt,
(b) der in der Verfahrensstufe (a) erhaltenen Mischung einen monoklonalen Antikörper gemäß Anspruch 1 oder 2 zugibt, wodurch ein Antikörper/Antigen-Komplex gebildet wird, und
(c) die Menge an humaner pankreatischer Prophospholipase A₂ in der Probe ausgehend von dem Ergebnis der Messung der Menge von an den in der Verfahrensstufe (b) erhaltenen Komplex gebundenem Marker berechnet.

4. Hybridoma-Zelle, welche einen monoklonalen Antikörper gemäß Anspruch 1 oder 2 bildet.

5. Verfahren zur Herstellung eines monoklonalen Antikörpers, der humane pankreatische Prophospholipase A₂ in spezifischer Weise erkennt, wobei der Antikörper der von der Hybridoma-Zelle PR414 (FERM BP-2978) gebildete monoklonale Antikörper PR414 ist, bei dem man:
i) die Prophospholipase A₂ aus humaner pankreatischer Flüssigkeit extrahiert und reinigt,
ii) ein Tier mit der Prophospholipase A₂ immunisiert,
iii) Antikörper-bildende Zellen des Tieres isoliert und mit Myelom-Zellen fusioniert, um Hybridoma-Zellen zu erhalten,
iv) die Hybridoma-Zellen einem Screening nach Hybridoma-Zellen zuführt, welche den monoklonalen Antikörper bilden, und
v) den monoklonalen Antikörper aus den den monoklonalen Antikörper bildenden Hybridoma-Zellen isoliert.

6. Verfahren nach Anspruch 5, bei dem die Kreuzreaktivität des monoklonalen Antikörpers gegenüber humaner pankreatischer Phospholipase A₂ weniger als 1 % beträgt.

7. Verfahren zur Analyse der humanen pankreatischen Prophospholipase A₂, bei dem man
(a) einer Probe, die humanes pankreatisches Prophospholipase A₂-Analyt enthält, eine vorgeschriebene Menge an markierter humaner pankreatischer Prophospholipase A₂ zugibt,
(b) der in der Verfahrensstufe (a) erhaltenen Mischung einen nach dem Verfahren gemäß Anspruch 5 oder 6 gebildeten monoklonalen Antikörper zugibt, wodurch ein Antikörper/Antigen-Komplex gebildet wird, und
(c) die Menge an humaner pankreatischer Prophospholipase A₂ in der Probe ausgehend von dem Ergebnis der Messung der Menge von an den in der Verfahrensstufe (b) erhaltenen Komplex gebundenem Marker berechnet.

8. Verfahren zur Herstellung einer Hybridoma-Zelle, die den nach dem Verfahren gemäß Anspruch 5 oder 6 gebildeten monoklonalen Antikörper produziert, bei dem man
i) die Prophospholipase A₂ aus humaner pankreatischer Flüssigkeit extrahiert und reinigt,
ii) ein Tier mit der Prophospholipase A₂ immunisiert,
iii) Antikörper-bildende Zellen des Tieres isoliert und mit Myelom-Zellen fusioniert, um Hybridoma-Zellen zu erhalten, und
iv) die Hybridoma-Zellen einem Screening nach Hybridoma-Zellen zuführt, welche den monoklonalen Antikörper bilden.

## Revendications (Revendications pour l'(les) Etat(s) contractant(s) suivant(s): AT, BE, DK, FR, DE, NL, IT, LU, SE, CH, LI, GB.)

1. Anticorps monoclonal reconnaissant spécifiquement la prophospholipase A₂ pancréatique humaine, dans lequel ledit anticorps est l'anticorps monoclonal PR414 produit par l'hybridome PR414 (FERM BP-2978).

2. Anticorps monoclonal selon la revendication 1, dans lequel la réactivité croisée dudit anticorps monoclonal avec la phospholipase A₂ pancréatique humaine est inférieure à 1%.

3. Procédé pour le dosage de la prophospholipase A₂ pancréatique humaine, comprenant les étapes consistant:
(a) à ajouter une quantité déterminée de prophospholipase A₂ pancréatique humaine à un échantillon contenant un analyte prophospholipase A₂ pancréatique humaine,
(b) à ajouter un anticorps monoclonal selon l'une des revendications 1 ou 2 au mélange obtenu dans l'étape (a), formant ainsi un complexe anticorps-antigène, et
(c) à calculer la quantité de prophospholipase A₂ pancréatique humaine dans ledit échantillon à partir du résultat de la mesure de la quantité de produit marqué lié dans le complexe obtenu dans l'étape (b).

4. Hybridome qui produit un anticorps monoclonal selon l'une des revendications 1 ou 2.

## Revendications (Revendications pour l'(les) Etat(s) contractant(s) suivant(s): ES, GR)

1. Anticorps monoclonal reconnaissant spécifiquement la prophospholipase A₂ pancréatique humaine, dans lequel ledit anticorps est l'anticorps monoclonal PR414 produit par l'hybridome PR414 (FERM BP-2978).

2. Anticorps monoclonal selon la revendication 1, dans lequel la réactivité croisée dudit anticorps monoclonal avec la phospholipase A₂ pancréatique humaine est inférieure à 1%.

3. Procédé pour le dosage de la prophospholipase A₂ pancréatique humaine, comprenant les étapes consistant:
(a) à ajouter une quantité déterminée de prophospholipase A₂ pancréatique humaine à un échantillon contenant un analyte prophospholipase A₂ pancréatique humaine,
(b) à ajouter un anticorps monoclonal selon l'une des revendications 1 ou 2 au mélange obtenu dans l'étape (a), formant ainsi un complexe anticorps-antigène, et
(c) à calculer la quantité de prophospholipase A₂ pancréatique humaine dans ledit échantillon à partir du résultat de la mesure de la quantité de produit marqué lié dans le complexe obtenu dans l'étape (b).

4. Hybridome qui produit un anticorps monoclonal selon l'une des revendications 1 ou 2.

5. Procédé pour la production d'un anticorps monoclonal reconnaissant spécifiquement la prophospholipase A₂ pancréatique humaine, dans lequel ledit anticorps est l'anticorps monoclonal PR414 produit par l'hybridome PR414 (FERM BP-2978), comprenant les étapes consistant:
(i) à extraire et à purifier la prophospholipase A₂ pancréatique provenant de suc pancréatique humain;
(ii) à immuniser un animal avec ladite prophospholipase A₂;
(iii) à isoler et à fusionner les cellules produisant l'anticorps dudit animal avec des cellules de myélome pour obtenir des hybridomes;
(iv) à cribler lesdits hybridomes pour isoler des hybridomes qui produisent ledit anticorps monoclonal; et
(v) à isoler ledit anticorps monoclonal desdits hybridomes qui produisent ledit anticorps monoclonal.

6. Procédé selon la revendication 5, dans lequel la réactivité croisée dudit anticorps monoclonal avec la phospholipase A₂ pancréatique humaine est inférieure à 1%.

7. Procédé pour le dosage de la prophospholipase A₂ pancréatique humaine, comprenant les étapes consistant:
(a) à ajouter une quantité déterminée de prophospholipase A₂ pancréatique humaine marquée à un échantillon contenant un analyte prophospholipase A₂ pancréatique humaine,
(b) à ajouter ledit anticorps monoclonal produit par le procédé selon l'une des revendications 5 ou 6 au mélange obtenu dans l'étape (a), formant ainsi un complexe anticorps-antigène, et
(c) à calculer la quantité de prophospholipase A₂ pancréatique humaine dans ledit échantillon d'après le résultat de la mesure de la quantité de produit marqué lié dans le complexe obtenu dans l'étape (b).

8. Procédé pour la production d'un hybridome qui produit l'anticorps monoclonal produit par le procédé selon l'une des revendications 5 ou 6 comprenant les étapes consistant:
i) à extraire et à purifier la prophospholipase A₂ à partir de suc pancréatique humain;
ii) à immuniser un animal avec ladite prophospholipase A₂;
iii) à isoler et à fusionner les cellules produisant l'anticorps dudit animal avec des cellules de myélome pour obtenir des hybridomes; et
iv) à cribler lesdits hybridomes pour isoler des hybridomes qui produisent ledit anticorps monoclonal.
